# FASCICULE DE BREVET EUROPEEN

(11) **EP 1 791 500 B1**
(45) Date de publication et mention de la délivrance du brevet: **04.06.2014**
(21) Numéro de dépôt: 05800676.8
(22) Date de dépôt: 07.09.2005
(51) Int. Cl.: A61F 2/24

(54) **VALVE PROTHETIQUE INTERCHANGEABLE**
AUSTAUSCHBARE KLAPPENPROTHESE
INTERCHANGEABLE PROSTHETIC VALVE

(30) Priorité: 07.09.2004 FR 0409468
(43) Date de publication de la demande: 06.06.2007
(73) Titulaire: Laboratoires Perouse, 60173 Ivry Le Temple (FR)
(72) Inventeur: STYRC, Mikolaj, Witold, L-8190 Kopstal (LU)
(74) Mandataire: Blot, Philippe Robert Emile
(86) Numéro de dépôt international: PCT/FR2005/002229
(87) Numéro de publication internationale: WO 2006/027500

(56) Documents cités:
- WO-A-98/11847
- WO-A-02/087467
- WO-A-03/030776
- WO-A1-2006/009690
- US-A1- 2004 019 374
- US-A1- 2004 098 112

## Description

La présente invention concerne une valve prothétique selon le préambule de la revendication 1.

Le coeur comporte deux oreillettes et deux ventricules qui sont séparés par des valves. Des valves sont en outre présentes en sortie du ventricule droit (valve pulmonaire) et du ventricule gauche (valve aortique).

Ces valves assurent une circulation univoque du flux sanguin, évitant un reflux sanguin à l'issue de la contraction ventriculaire.

Des maladies affectent les valves. En particulier, celles-ci peuvent souffrir d'une mauvaise ouverture, réduisant ainsi le flux sanguin, ou n'être que partiellement étanches, autorisant ainsi un reflux ou une régurgitation vers le ventricule ayant expulsé le flux sanguin.

Ces problèmes de régurgitation conduisent à une dilatation anormale du ventricule qui produit, à terme, une insuffisance cardiaque.

Il est connu de traiter ce type de maladie de manière chirurgicale, en remplaçant la valve malade. Les valves malades, et notamment la valve aortique en sortie du ventricule gauche sont remplacées par une valve prélevée sur un sujet décédé, ou par une valve prothétique couramment désignée par bioprothèse. Une valve prothétique est constituée d'une armature métallique annulaire et d'un obturateur souple réalisé dans un tissu d'origine animale. L'obturateur est solidarisé à demeure sur l'armature.

De telles valves sont décrites, notamment dans les documents WO-01/03095 et WO-00/27975.

Une fois implantée, l'armature s'applique contre la paroi intérieure du coeur à laquelle elle est cousue, notamment en entrée de l'artère aortique issue du ventricule gauche.

On constate, après plusieurs années d'implantation d'une telle prothèse, que celle-ci se dégrade et qu'elle ne fonctionne plus de manière efficace. En particulier, l'obturateur souple se déchire et présente des trous, ou l'obturateur se calcifie perdant ainsi sa souplesse et n'étant alors plus à même de se déformer pour exercer sa fonction de valve. Une nouvelle prothèse doit alors être mise en place.

Toutefois, le retrait de l'ancienne prothèse ne peut être effectué de manière endoluminale, notamment du fait que l'armature porteuse de la prothèse est cousue avec la paroi cardiaque, interdisant leur désolidarisation sans une intervention chirurgicale lourde prévoyant le remplacement complet de la valve.

Afin d'éviter une intervention chirurgicale lourde pour retirer l'ancienne prothèse et chercher à en fixer une seconde, il a été envisagé de disposer, par voie endoluminale, une nouvelle valve prothétique à l'intérieur de l'ancienne prothèse laissée en place.

La nouvelle valve prothétique est formée d'un support tubulaire en treillis déformable radialement équipé d'un obturateur souple disposé dans le conduit délimité par le support tubulaire. Cet obturateur est lié au support tubulaire et présente une forme telle qu'il soit propre, par déformation, à autoriser la circulation du sang dans un sens et à bloquer la circulation du sang, dans le sens opposé.

Des valves prothétiques comportant un support tubulaire en treillis sont décrites par exemple dans WO-A-03/030776 et dans WO-A-2006/009690, ce dernier document faisant partie de l'état de la technique au titre de l'article 54(3) CBE.

Il a été envisagé que le support tubulaire soit formé de fils métalliques élastiques entrelacés délimitant des mailles généralement en forme de losanges. Un tel support tubulaire est connu sous le terme de "stent". Ce support tubulaire est déformable entre une position de mise en place dans laquelle son diamètre est réduit et une position d'implantation dans laquelle son diamètre est plus important et est suffisant pour que le support s'appuie à l'intérieur de l'armature porteuse de l'ancienne prothèse.

Pour leur mise en place, de telles valves prothétiques comportant un support tubulaire en treillis sont disposées dans un cathéter de petit diamètre. L'extrémité du cathéter est amenée au travers du réseau artériel jusqu'à la région équipée de l'ancienne prothèse ne fonctionnant plus. La nouvelle valve prothétique est poussée hors du cathéter à l'aide d'un organe filiforme engagé dans le cathéter. Le support tubulaire étant élastique, celui-ci se déploie immédiatement et de manière autonome lorsqu'il n'est plus comprimé radialement par le cathéter. Il vient alors s'appliquer sur le pourtour intérieur de l'armature porteuse de l'ancienne prothèse.

La nouvelle valve prothétique est mise en place alors que le coeur fonctionne toujours. Dans le cas du traitement d'une valve aortique, la valve prothétique est amenée à contre courant du flux sanguin. Ainsi, lors du largage de la nouvelle valve prothétique, celle-ci se déploie à l'entrée de l'artère aortique et obstrue alors celle-ci. Lors de son déploiement, la nouvelle valve prothétique présente une surface transversale importante. Ainsi, lors d'une contraction cardiaque conduisant à une expulsion de sang dans l'aorte, la valve prothétique risque de se trouver entraînée lors de son déploiement et peut donc se trouver positionnée en dehors de l'armature porteuse de l'ancienne valve. La nouvelle valve prothétique obstrue alors l'artère sans remplir sa fonction de manière satisfaisante.

Les conséquences d'une mauvaise mise en place de la nouvelle valve prothétique sont souvent très dommageables pour le patient puisque la valve prothétique nouvellement introduite ne peut être retirée autrement que par voie chirurgicale.

Pour éviter cette difficulté, il est connu de larguer la nouvelle valve prothétique rapidement et exactement entre deux contractions cardiaques. Toutefois, ce lapse de temps étant très court, la mise en place de la nouvelle valve prothétique est délicate.

L'invention a pour but de proposer une valve prothétique pouvant être mise en place par voie endoluminale, sans risque important de mauvais positionnement axial, même en présence d'un flux sanguin puissant dans la région d'implantation.

A cet effet, l'invention a pour objet une valve prothétique interchangeable selon la revendication 1.

Suivant des modes particuliers de réalisation, la valve prothétique comporte l'une ou plusieurs des caractéristiques des revendications 2 à 9.

L'invention a, en outre pour objet, un nécessaire de traitement selon la revendication 10.

L'invention sera mieux comprise à la lecture de la description qui va suivre, donnée uniquement à titre d'exemple et faite en se référant aux dessins, sur lesquels :
- la figure 1 est une vue en perspective d'une valve prothétique implantée de manière chirurgicale qui est endommagée ;
- la figure 2 est une vue en perspective d'une valve prothétique selon l'invention dans son état obturant, la valve étant en cours d'implantation au travers d'une ancienne valve prothétique endommagée ;
- la figure 3 est une vue en bout de la valve prothétique de la figure 2 ;
- la figure 4 est une vue identique à celle de la figure 3, la valve prothétique étant dans son état passant ;
- les figures 5 et 6 sont des vues en coupe longitudinale illustrant les stades successifs de mise en place d'une valve prothétique selon l'invention ;
- les figures 7 et 8 sont des vues identiques à celles des figures 5 et 6 illustrant des stades successifs de retrait d'une valve prothétique selon l'invention.

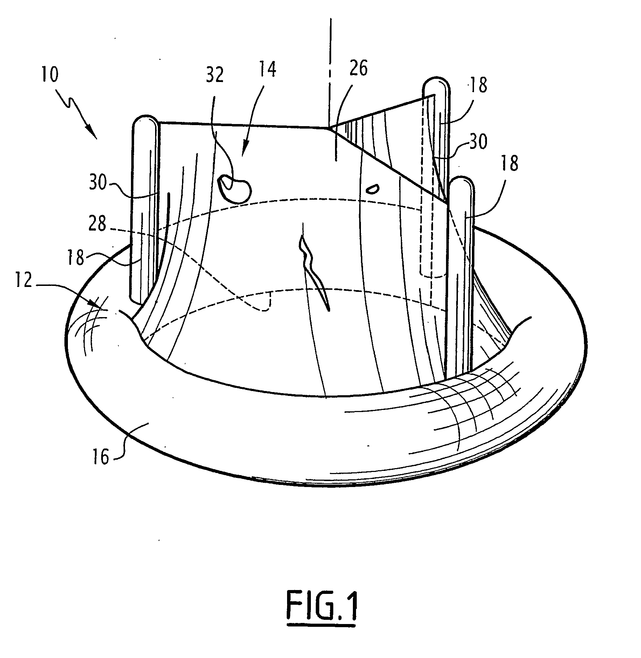

Sur la figure 1 est représentée une valve prothétique 10 endommagée et qui doit être traitée. Cette valve prothétique est supposée avoir été implantée chirurgicalement, par exemple pour remplacer une valve aortique du coeur. Ainsi, cette valve est placée immédiatement en amont de l'aorte à l'emplacement de la valve naturelle.

Une telle valve prothétique est connue en soi et comporte essentiellement une armature porteuse 12 et un obturateur souple 14.

L'armature porteuse 12 comporte essentiellement une embase annulaire rigide 16 portant trois plots 18 rigides s'étendant chacun parallèlement à l'axe de l'embase annulaire 16. Cette embase est formée d'un tore métallique rigide auquel sont soudés les trois plots 18. Le tore est enveloppé sur toute sa surface d'une nappe tissée permettant la solidarisation de la valve prothétique au tissu organique du coeur par suture entre la nappe tissée et la paroi organique du coeur. Le diamètre intérieur de l'embase annulaire 16 est compris entre 15 mm et 40 mm.

Les plots 18 sont liés, depuis une extrémité, à l'embase annulaire 16 et font tous saillie du même côté. Ils sont régulièrement répartis angulairement autour de l'axe de l'armature porteuse 12. La hauteur totale des plots 18 y compris l'embase annulaire 16 est comprise entre 10 mm et 30 mm.

L'obturateur souple 14 est lié à demeure è la fois aux plots 18 et l'embase annulaire 16. Dans le mode de réalisation illustré, l'obturateur souple est formé de trois membranes 26 généralement de forme rectangulaire. Suivant un grand côté 28 formant base, chaque membrane 26 est reliée à l'armature porteuse entre deux plots 18 successifs. Ainsi, suivant cette base, la membrane décrit un arc de cercle le long de l'embase annulaire 16. Les deux bords latéraux de la membrane sont liés, suivant la longueur des plots 18.

Comme connu en soi, les trois membranes 26 formant l'obturateur souple sont normalement déformables entre une position d'obturation dans laquelle les bords libres des membranes sont accolés les uns aux autres, les membranes délimitant extérieurement trois poches d'accumulation du sang en étant déformées vers l'axe de la valve prothétique, et une position de passage dans laquelle les trois membranes sont écartées les unes des autres et s'étendent généralement dans le prolongement axial de l'embase annulaire, les trois membranes délimitant alors ensemble un passage généralement cylindrique permettant la circulation du flux sanguin.

Telle qu'illustrée sur la figure 1, la valve prothétique 10 est endommagée par des trous 32 formés dans les membranes 26, ces trous conduisant à une mauvaise étanchéité de la valve.

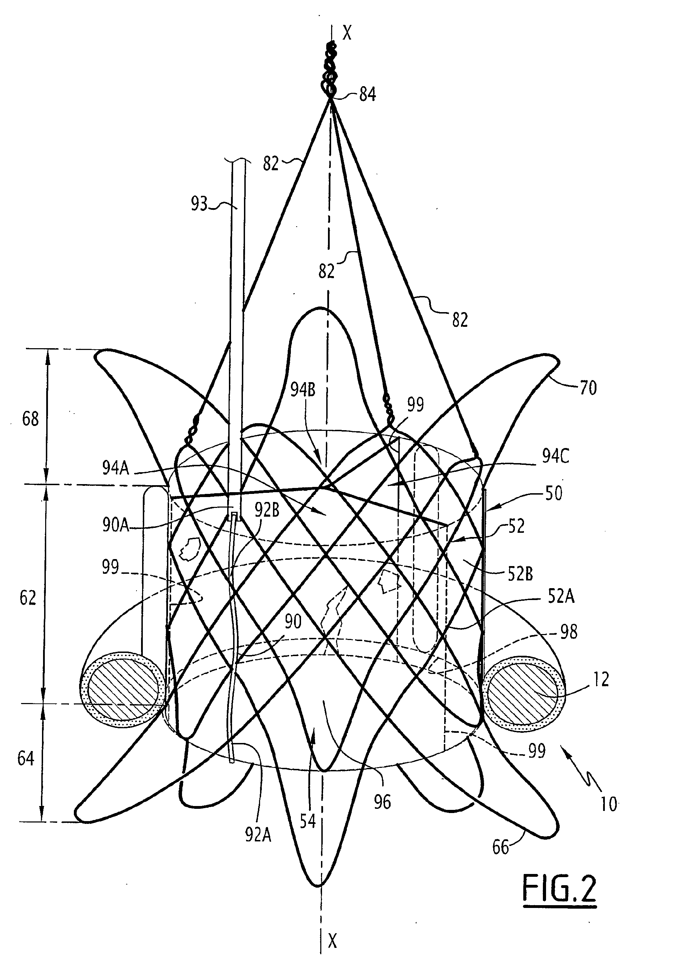

Sur la figure 2 est représentée une valve prothétique 50 selon l'invention après mise en place par voie endoluminale à l'intérieur de la valve prothétique endommagée 10 préalablement implantée par voie chirurgicale.

Ainsi, la valve prothétique endommagée 10 est visible sur cette figure et une valve prothétique 50 selon l'invention est engagée dans l'armature porteuse 12 de la valve prothétique endommagée.

La valve prothétique 50 comporte un support tubulaire en treillis 52 d'axe X-X et un obturateur souple 54 lié au support tubulaire 52 et disposé l'intérieur de celui-ci.

La valve 50 est remplaçable et est notamment amovible par rapport à la valve endommagée 10.

Le support tubulaire 52 est constitué, par exemple, d'un treillis tubulaire 52A noyé dans un film 52B extensible et étanche aux liquides tel qu'un élastomère. Le film 52B recouvrant le treillis, définit suivant toute la hauteur du support 52 une paroi cylindrique pleine, étanche aux liquides. Le treillis 52A est constitué d'acier inoxydable ayant des propriétés élastiques, de sorte que le support 52 est auto-expansible. Un tel support lorsqu'il est utilisé seul est couramment désigné par le terme anglais "stent".

Comme connu en soi, le support 52 est susceptible de se déformer spontanément d'un état comprimé, dans lequel il présente un petit diamètre à un état dilaté, dans lequel il présente un diamètre supérieur, cet état dilaté constituant son état de repos.

Dans son état implanté, tel qu'illustré sur les figures 2 à 4, le support 52 s'applique, du fait de son élasticité, contre l'embase annulaire 16 et les plots 18 de la valve endommagée 10, en maintenant les trois membranes 26 appliquées sur la surface externe du support 52.

A chacune de ses extrémités axiales, le support 52 se prolonge axialement au-delà de l'armature porteuse de la valve endommagée 10 par deux collerettes divergentes présentant une forme généralement tronconique s'évasant vers les extrémités axiales du support.

Plus précisément, le support 52 présente un tronc médian 62 généralement cylindrique dont la longueur correspond à la hauteur de l'armature porteuse de la valve endommagée, cette hauteur étant mesurée suivant l'axe de la valve. La hauteur du tronc est comprise entre 10 mm et 30 mm.

Le treillis délimitant le tronc 62 est forme de fils métalliques entrelacés. Ainsi, deux familles de fils s'entrecroisent. Les fils de la première famille décrivent des hélices orientées dans un même sens et s'étendant généralement parallèlement les unes aux autres. Au contraire, les fils de la seconde famille décrivent des hélices orientées en sens inverse et s'étendant parallèlement les unes aux autres. Les fils des première et seconde familles sont engagés successivement au-dessus et au-dessous les uns des autres, de sorte que ces familles de fils délimitent des mailles en forme de losanges, dont une diagonale de chaque maille s'étend suivant l'axe du support, et dont l'autre diagonale s'étend généralement perpendiculairement.

A une première extrémité du support, le tronc 62 est prolongé par une collerette évasée 64 formée d'un ensemble de lobes 66 s'écartant de l'axe du support en direction de leur extrémité recourbée. Ces lobes sont formés de boucles formées aux extrémités des fils des première et seconde familles, lesquels sont venus de matière.

De même, à son autre extrémité, le support comporte une seconde collerette évasée 68 prolongeant le tronc 62. Celle-ci est également délimitée par des lobes 70 déformés vers l'extérieur.

Au repos, l'extrémité libre des collerettes, c'est-à-dire la section extrême la plus évasée de ces collerettes, définit un contour dont le diamètre est égal au diamètre du tronc 62 augmenté de 5 mm à 15 mm.

De même, avantageusement, la hauteur des collerettes 64, 68 mesurée suivant l'axe du support tubulaire 52 est comprise entre 5 mm et 15 mm et est par exemple égale à 10 mm.

Le film 52B dans lequel est noyé le treillis tubulaire 52A se prolonge sur les lobes formant les collerettes 64 et 68.

Suivant un premier mode de réalisation, le support tubulaire 52 présente sur toute sa hauteur au repos, lorsqu'il n'est pas comprimé dans une armature 12, un diamètre supérieur au diamètre de l'armature 12, de sorte que les collerettes 64 et 68 se mettent en forme évasée du seul fait de l'élasticité naturelle du support tubulaire alors que le tronc est maintenu confiné sous forme tubulaire dans l'armature porteuse 12 de la valve prothétique endommagée.

En variante, le tronc 62 du support tubulaire présente, au repos, même lorsqu'il n'est pas comprimé dans une armature 12, un diamètre inférieur au diamètre d'extrémité des collerettes 64 et 68.

Par ailleurs, trois paires de fils issus des première et seconde familles respectivement se relient par paire l'un à l'autre au niveau de la collerette 68 pour former trois jambes 82. Les jambes convergent l'une vers l'autre suivant l'axe X-X de la valve prothétique pour se réunir en un point de liaison 84 contenu sur cet axe. Les trois jambes 82 délimitent ainsi un trépied. Elles sont angulairement régulièrement réparties autour de l'axe X-X et délimitent chacune avec cet axe un angle compris entre 20° et 40°. Pour leur liaison, les trois jambes 82 sont par exemple torsadées ensemble au point 34. Une boucle d'accrochage est formée au point extrême 34.

En outre, et selon l'invention, le support tubulaire 52 comporte au moins une membrure rigide 90 s'étendant généralement suivant une génératrice du support tubulaire 52. Cette membrure est liée au support en au moins deux points 92A, 92B espacés suivant l'axe du support. Ces deux points sont formés sur la hauteur du tronc 62, notamment au voisinage de la région de liaison avec les collerettes 64 et 68. La liaison est effectuée par soudage ou collage.

Avantageusement, une unique membrure 90 est formée suivant une génératrice du tronc 62. Cette membrure est constituée par exemple d'un fil métallique rigide longitudinalement qui est engagé au travers des mailles du treillis alternativement à l'intérieur et à l'extérieur du treillis.

Avantageusement, les extrémités de la membrure sont disposées à l'intérieur du support tubulaire, c'est-à-dire du côté de l'axe X-X par rapport au film étanche 52B.

Une extrémité en saillie 90A de la membrure 90 au moins, et notamment celle du côté des jambes 82 est propre à coopérer avec un tuteur 93 pour assurer leur solidarisation axiale, comme illustré sur la figure 2 et comme cela sera expliqué ultérieurement. La solidarisation axiale entre le tuteur 93 et la membrure 90 est assurée, par exemple, par emboîtement de l'extrémité de liaison 90A de la membrure dans un logement prévu dans l'épaisseur du tuteur 93 et débouchant au bout de celui-ci.

L'obturateur 54 est relié à la surface intérieure du support tubulaire 52. Cet obturateur est formé de trois membranes souples 94A, 94B, 94C agencées comme l'obturateur 14 de la valve prothétique 10. Ainsi, chaque membrane 94A, 94B, 94C est formée d'un film de polymère ou d'une couche de film organique telle que du péricarde de veau. Chaque membrane est généralement de forme rectangulaire. Elle est liée à la surface interne du film étanche 52B suivant un grand côté 98 formant base suivant la circonférence de liaison entre le tronc 62 et la collerette élargie 64.

Les bords longitudinaux 99 des trois membranes 94A, 94B, 94C, sont liés au support tubulaire 52 suivant trois génératrices de celui-ci réparties régulièrement de manière angulaire autour de l'axe du support tubulaire. Ainsi, les membranes sont liées deux à deux suivant leurs bords longitudinaux sur le support tubulaire. Cette liaison s'effectue sur toute la hauteur du tronc 62.

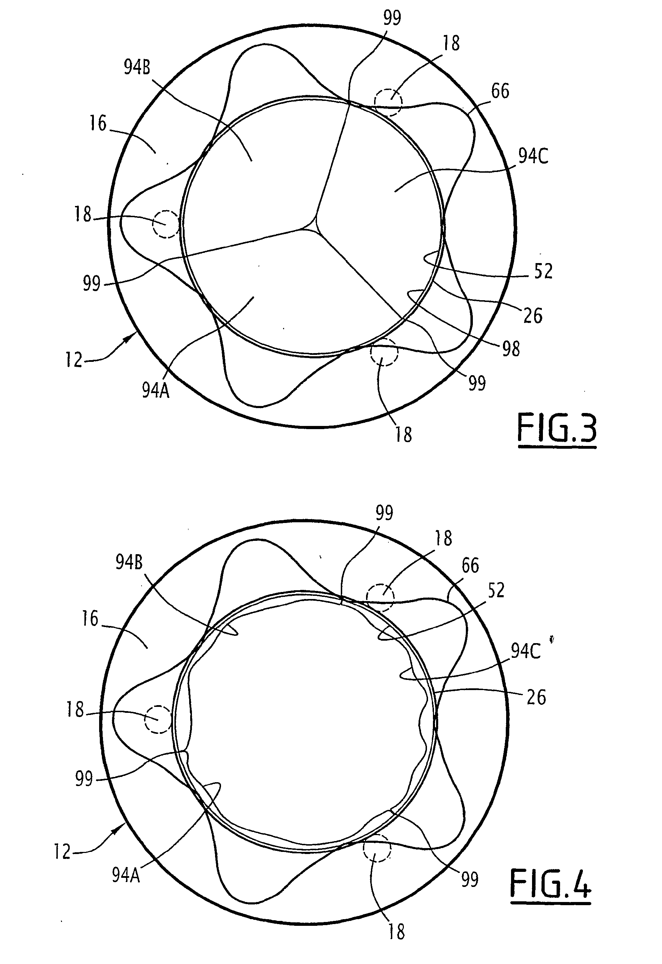

Les membranes 94A, 94B, 94C formant l'obturateur sont déformables entre une position d'obturation représentée sur les figures 2 et 3 dans laquelle les bords libres des membranes sont accolées deux à deux par moitié et une position de passage du flux sanguin illustrée sur la figure 4 dans laquelle les trois membranes sont écartées les unes des autres.

Dans la position d'obturation, les trois membranes délimitent, avec la paroi tubulaire du support 52, trois poches de retenue du flux sanguin. Au contraire, dans la position de passage, les trois membranes sont appliquées sur la surface intérieure du support tubulaire, comme illustré sur la figure 4, délimitant ainsi ensemble un conduit généralement circulaire pour la circulation du flux sanguin.

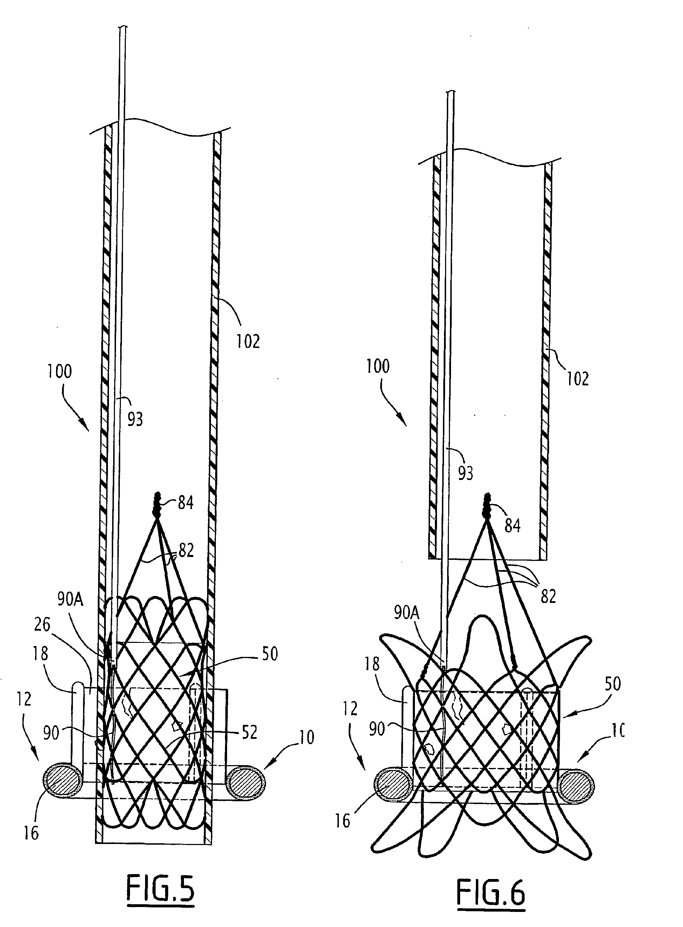

Pour le traitement de la valve prothétique endommagée, la nouvelle valve prothétique est mise en place dans l'espace délimité par l'armature porteuse 12 de la valve endommagée, comme illustré sur les figures 5 et 6.

A cet effet, un nécessaire de traitement 100 illustre sur ces figures est mis en oeuvre. Il comporte une nouvelle valve prothétique 50 contenue dans un cathéter 102 de diamètre extérieur inférieur au diamètre intérieur de l'armature porteuse 12.

Comme illustré sur la figure 5, la valve prothétique, et notamment le support tubulaire 52, est comprimé radialement à l'intérieur du tube.

En outre le tuteur 93 s'étend suivant la longueur du cathéter 102 en étant solidarisé depuis son extrémité à l'extrémité de la membrure de rigidification axiale 90. Le tuteur 93 présente axialement une rigidité suffisante pour pousser la valve prothétique hors du cathéter 102.

Pour la mise en place, l'extrémité du cathéter 102 dans lequel est logée la valve prothétique est introduite dans l'aorte du patient puis est déplacée progressivement dans l'aorte jusqu'à l'emplacement de la valve prothétique endommagée en sortie du coeur. Le déplacement du cathéter s'effectue alors à contre-courant du flux sanguin normal.

Le cathéter est amené dans la position illustrée sur la figure 5. Dans cette position, le cathéter 102 est alors tiré alors que la nouvelle valve prothétique 50 est maintenue en place par le tuteur 93. Au fur et à mesure du déplacement du cathéter 102, la valve prothétique 50 se trouve découverte, de sorte que son extrémité se déploie pour former la collerette 64 puis le tronc 62 de support tubulaire vient s'appliquer contre les plots 18 et enfin son autre extrémité se déploie pour former la collerette 68.

Lors de la mise à nu progressive de la valve prothétique 50 par déplacement du cathéter 102, la valve prothétique est maintenue fixe axialement par rapport au conduit aortique et notamment par rapport à l'ancienne valve prothétique 10 endommagée grâce au tuteur rigide 93 qui retient dans son prolongement la membrure 90. Ainsi, la présence du tuteur 93 coopérant avec la membrure 90 réduit les risques de déplacement axial de la valve prothétique pendant son largage, même si celle-ci est larguée lors d'une pulsation cardiaque conduisant à une mise en circulation du sang à l'endroit d'implantation de la valve.

Après déploiement, la valve est maintenue axialement par la présence des collerettes élargies 64 et 68 s'appuyant respectivement sur l'embase annulaire 16 et l'extrémité des plots 18.

Après largage, le tuteur 93 est retiré par simple traction. Ainsi, la membrure 90 se désemboîte de l'extrémité du tuteur 93. La membrure 90 reste en place puisqu'elle est intégrée au support tubulaire 52.

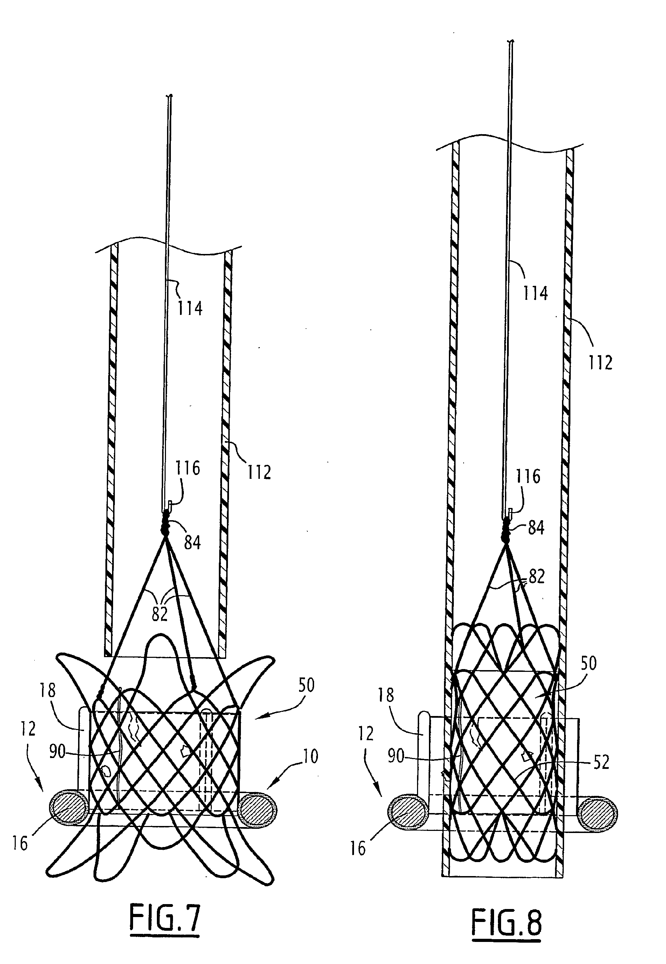

Comme illustré sur les figures 7 et 8, pour le retrait de la valve prothétique 50, un cathéter 112 est introduit au travers de l'aorte et est disposé en regard de l'extrémité de la valve présentant le trépied formé des jambes 82.

Un outil de traction 114 est acheminé au travers du cathéter 112. Cet outil comporte, à son extrémité, une crosse 116 permettant d'accrocher le point de liaison 84 du trépied. Alors que l'extrémité ouverte du cathéter est en contact avec les jambes 82 du trépied, la valve prothétique 50 est progressivement introduite à l'intérieur du cathéter 112 en avançant le cathéter 112 suivant la longueur de la valve 50. Par effet de came, les jambes 82 sont resserrées vers l'axe et la valve prothétique est progressivement amenée dans son état resserré et introduite dans le cathéter 112, comme illustré sur la figure 8. Le cathéter 112 renfermant la valve prothétique 50 est alors extrait du corps humain.

Une nouvelle valve prothétique 50 est alors introduite à l'aide d'un nécessaire de traitement 100 dans le corps humain et la valve est larguée comme exposé précédemment.

## Revendications

1. Valve prothétique (50) pour mise en place par voie endoluminale comportant:
- un support tubulaire (52) déformable radialement par rapport à un axe principal (X-X) entre une position déployée d'implantation et une position repliée de mise en place ;
- un obturateur souple (54) lié au support tubulaire (52) et déformable entre une position d'obstruction dans laquelle il est étendu transversalement et une position de libération dans laquelle il est contracté transversalement sous l'action du flux circulant au travers du support tubulaire (52),
**caractérisée en ce qu'**elle comporte au moins une membrure rigide (90) s'étendant généralement suivant une génératrice du support tubulaire (52), laquelle membrure (90) est liée au support tubulaire (52) en au moins deux points (92A, 92B) espacés suivant l'axe du support tubulaire (52), la liaison entre la membrure (90) et le support tubulaire (52) étant effectuée par soudage ou par collage.

2. Valve prothétique (50) selon la revendication 1, **caractérisée en ce que** le support tubulaire (52) délimite une paroi cylindrique pleine, étanche aux liquides.

3. Valve prothétique (50) selon la revendication 2, **caractérisée en ce que** le support tubulaire (52) comporte un treillis tubulaire (52A) recouvert d'un film (52B) extensible et étanche aux liquides formant ladite paroi cylindrique pleine.

4. Valve prothétique (50) selon la revendication 3, **caractérisée en ce que** la ou chaque membrure (90) est engagée alternativement dans les mailles du treillis (52A).

5. Valve prothétique (50) selon l'une quelconque des revendications précédentes, **caractérisée en ce que** le support tubulaire (52) présente un tronc médian (62) généralement cylindrique et, axialement de part et d'autre du tronc (62), deux collerettes (66, 70) généralement tronconiques s'évasant depuis le tronc (62) vers les extrémités du support (52).

6. Valve prothétique (50) selon l'une quelconque des revendications précédentes, **caractérisée en ce que** le support tubulaire (52) est élastique et est conformé pour être sollicité élastiquement de sa position repliée à sa position déployée.

7. Valve prothétique (50) selon l'une quelconque des revendications précédentes, **caractérisée en ce que** la ou chaque membrure (90) comporte une extrémité en saillie (90A) de liaison à un tuteur (93) de maintien de la valve prothétique (50).

8. Valve prothétique (50) selon l'une quelconque des revendications précédentes, **caractérisée en ce que** le support tubulaire (52) est prolongé par des jambes convergentes (82) formant trépied, lesquelles jambes sont liées entre elles en un point de liaison (84) contenu sensiblement sur l'axe (X-X) du support tubulaire (52).

9. Valve prothétique (50) selon l'une quelconque des revendications précédentes, **caractérisée en ce que** l'obturateur (50) comporte trois membranes (94A, 94B, 94C) déformables entre une position d'obturation dans laquelle les bords libres des membranes sont accolés deux à deux par moitié et une position de passage du flux sanguin dans laquelle les trois membranes sont écartées les unes des autres.

10. Nécessaire de traitement (100) comportant :
○ une valve prothétique (50) selon l'une quelconque des revendications précédentes ;
○ un cathéter (102) de mise en place de la valve; et
○ un tuteur (104) de retenue de la valve prothétique, lequel tuteur comporte des moyens de liaison axiale du tuteur dans le prolongement de la membrure (90) de la valve prothétique (50).

## Patentansprüche

1. Klappenprothese (50) für Platzierung auf endoluminalem Weg, die aufweist:
- eine im Verhältnis zu einer Hauptachse (X-X) zwischen einer entfalteten Implantationsstellung und einer zusammengefalteten Platzierungsstellung radial verformbare röhrenförmige Stütze (52),
- einen mit der röhrenförmigen Stütze (52) verbundenen flexiben Verschluss (54), der zwischen einer Verschlussstellung, in der er transversal ausgebreitet ist, und einer Freigabestellung, in der er unter der Wirkung des durch die röhrenförmige Stütze (52) fließenden Stroms transversal zusammengezogen ist, verformbar ist,
**dadurch gekennzeichnet, dass** sie mindestens eine starre Gurtung (90) aufweist, die sich im Allgemeinen gemäß einer Mantellinie der röhrenförmigen Stütze (52) erstreckt, wobei die Gurtung (90) an mindestens zwei Punkten (92A, 92B), die gemäß der Achse der röhrenförmigen Stütze (52) beabstandet sind, mit der röhrenförmigen Stütze (52) verbunden ist, wobei die Verbindung zwischen der Gurtung (90) und der röhrenförmigen Stütze (52) durch Schweißen oder Kleben erfolgt ist.

2. Klappenprothese (50) nach Anspruch 1, **dadurch gekennzeichnet, dass** die röhrenförmige Stütze (52) eine flüssigkeitsdichte volle zylindrische Wand begrenzt.

3. Klappenprothese (50) nach Anspruch 2, **dadurch gekennzeichnet, dass** die röhrenförmige Stütze (52) ein röhrenförmiges Gitterwerk (52A) aufweist, das mit einer dehnbaren und flüssigkeitsdichten Folie (52B) bedeckt ist, die die volle zylindrische Wand bildet.

4. Klappenprothese (50) nach Anspruch 3, **dadurch gekennzeichnet, dass** die oder jede Gurtung (90) alternierend in die Maschen des Gitterwerks (52A) eingreift.

5. Klappenprothese (50) nach einem der vorangehenden Ansprüche, **dadurch gekennzeichnet, dass** die röhrenförmige Stütze (52) einen allgemein zylindrischen Mittelrumpf (62) und axial auf der einen und der anderen Seite des Rumpfs (62) zwei allgemein kegelstumpfförmige Halskrausen (66, 70) aufweist, die sich ab dem Rumpf (62) in Richtung der Enden der Stütze (52) erweitern.

6. Klappenprothese (50) nach einem der vorangehenden Ansprüche, **dadurch gekennzeichnet, dass** die röhrenförmige Stütze (52) elastisch ist und ausgebildet, um von ihrer zusammengefalteten Stellung in ihre entfalteten Stellung elastisch beansprucht zu werden.

7. Klappenprothese (50) nach einem der vorangehenden Ansprüche, **dadurch gekennzeichnet, dass** die oder jede Gurtung (90) ein zur Verbindung der Gurtung an einem Halter (93) der Klappenprothese (50) hervorstehendes Verbindungsende (90A) aufweist.

8. Klappenprothese (50) nach einem der vorangehenden Ansprüche, **dadurch gekennzeichnet, dass** die röhrenförmige Stütze (52) durch konvergierende Schenkel (82), die einen Dreifuß bilden, verlängert wird, wobei die Schenkel miteinander in einem Verbindungspunkt (84) verbunden sind, der etwa auf der Achse (X-X) der röhrenförmigen Stütze (52) enthalten ist.

9. Klappenprothese (50) nach einem der vorangehenden Ansprüche, **dadurch gekennzeichnet, dass** der Verschluss (50) drei Membranen (94A, 94B, 94C) aufweist, die zwischen einer Verschlussstellung, in der die freien Ränder der Membranen paarig hälftig aneinanderstoßen, und einer zum Durchgang des Blutstroms Durchgangsstellung, in der die drei Membranen voneinander entfernt sind, verformbar sind.

10. Behandlungsset (100), das aufweist:
• eine Klappenprothese (50) nach einem der vorangehenden Ansprüche,
• einen Katheter (102) zur Platzierung der Klappe, und
• einen Halter (104) zum Halten der Klappenprothese, wobei der Halter axiale Verbindungsmittel zur Verbindung des Halters in Verlängerung der Gurtung (90) der Klappenprothese (50) aufweist.

## Claims

1. Prosthetic valve (50) for being put into place by an endoluminal approach, the valve comprising:
• a tubular support (52) that is radially deformable relative to a main axis (X-X) between a deployed implantation position and a folded positioning position; and
• a flexible shutter (54) connected to the tubular support (52) and deformable between an obstruction position in which it extends transversally and a release position in which it is contracted transversally under the action of a flow of blood through the tubular support (52),
**characterized in that** it includes at least one rigid member (90) extending generally along a generator line of the tubular support (92), which member (90) is connected to the tubular support (52) at at least two points (92A, 92B) that are spaced along the axis of the tubular support (52), the connection between the member (90) and the tubular support (52) being performed by welding or by adhesive bonding.

2. Prosthetic valve (50) according to claim 1, **characterized in that** the tubular support (52) defines a solid cylindrical wall that is liquid-proof.

3. Prosthetic valve (50) according to claim 2, **characterized in that** the tubular support (52) comprises a tubular lattice (52A) covered in a stretchable film (52B) that is liquid-proof and that forms said solid cylindrical wall.

4. Prosthetic valve (50) according to claim 3, **characterized in that** the or each member (90) is engaged in alternation in the meshes of the lattice (52A).

5. Prosthetic valve (50) according to any preceding claim, **characterized in that** the tubular support (52) presents a generally cylindrical middle trunk (52) and, axially at each end of the trunk (52), two generally frustoconical collars (66, 70) flaring away from the trunk (62) towards the ends of the supports (52).

6. Prosthetic valve (50) according to any preceding claim, **characterized in that** the tubular support (52) is resilient and is shaped to be urged resiliently from its folded position towards its deployed position.

7. Prosthetic valve (50) according to any preceding claim, **characterized in that** the or each member (90) has a projecting end (90A) for connection to a prop (93) for holding the prosthetic valve (50) in place.

8. Prosthetic valve (50) according to any preceding claim, **characterized in that** the tubular support (52) is extended by converging legs (82) forming a tripod, which legs are connected to one another at a connection point (84) lying substantially on the axis (X-X) of the tubular support (52).

9. Prosthetic valve (50) according to any preceding claim, **characterized in that** the shutter (50) has three membranes (94A, 94B, 94C) that are deformable between a closed position in which the free edges of the membranes touch one another in pairs over half their length, and an open position for passing the flow of blood in which the three membranes are spaced apart from one another.

10. A treatment kit (100) comprising:
• a prosthetic valve (50) according to any preceding claim;
• a catheter (102) for putting the valve into place; and
• a prop (104) for holding the prosthetic valve, which prop includes means for interconnecting the prop in line with the member (90) of the prosthetic valve (50).
